# EUROPÄISCHE PATENTSCHRIFT

(11) **EP 0 455 075 B1**
(45) Veröffentlichungstag und Bekanntmachung des Hinweises auf die Patenterteilung: **06.04.1994**
(21) Anmeldenummer: 91106374.1
(22) Anmeldetag: 19.04.1991
(51) Int. Cl.: A61M 5/32

(54) **Gerät zum Unschädlichmachen von Kanülen**
Device to render canulae harmless
Dispositif pour rendre des canules inoffensives

(30) Priorität: 27.04.1990 DE 9004788 U
(43) Veröffentlichungstag der Anmeldung: 06.11.1991
(73) Patentinhaber: MePhaTec GmbH Medizin- und Pharmatechnik, D-52224 Stolberg (DE)
(72) Erfinder: Fladung, Rüdiger, W-3408 Duderstadt (DE)
(74) Vertreter: Lins, Edgar, Dipl.-Phys. Dr.jur.

(56) Entgegenhaltungen:
- EP-A- 0 136 392
- EP-A- 0 332 584
- GB-A- 2 211 420

## Beschreibung

Die Erfindung betrifft ein Gerät zum Unschädlichmachen von zum einmaligen Gebrauch bestimmten Kanülen einer Spritze, mit einem Gehäuse mit einer kreisförmig ausgebildeten Einführöffnung, die nur die Einführung des Metallrohres der Kanüle in dessen Längsrichtung erlaubt, und einer unterhalb der Einführöffnung angeordneten Elektrodenanordnung mit einer unmittelbar unterhalb der Einführöffnung angeordneten ersten Elektrode mit einer Durchtrittsöffnung für das Metallrohr und einer zweiten Elektrode, die als massiver Metallblock ausgebildet ist und auf die die Spitze des Metallrohres nach dem Durchtritt durch die erste Elektrode trifft und so einen Stromkreis zum Abschmelzen der jeweiligen Spitze des Metallrohres schließt, wobei das Metallrohr beim weiteren Einführen weiter abgeschmolzen wird und die Einführbewegung durch einen Anschlag der Einführöffnung für einen Kopf der Kanüle begrenzt wird.

Aus Gründen der Infektionsgefahr werden überwiegend Spritzen verwendet, die nur zum einmaligen Gebrauch bestimmt sind. Sie bestehen aus einem Kunststoff-Spritzenkörper, auf den die aus einem Metallrohr und einem einen Kanülenkopf bildenden Kunststoffkörper bestehende Kanüle aufgeschoben wird. Um zu verhindern, daß ein mehrfacher Gebrauch solcher Kanülen stattfindet, der die Gefahr von Infektionen mit sich bringt, ist es bekannt, die Kanüle bzw. ihr Metallrohr aus dem geraden Zustand manuell zu verbiegen und in eine gekrümmte Gestalt zu verformen, so daß ohne weiteres erkennbar ist, daß die Kanüle bereits einmal benutzt wurde. Die Kanülen gelangen in diesem Zustand nicht selten in den Hausmüll. Es ist aber auch bereits bekannt, die Kanülen nach der einmaligen Benutzung im geraden oder im verbogenen Zustand in einen Sonderbehälter zu werfen, der aus einem eimerähnlichen Gehäuse besteht, der dann nach entsprechender Füllung mit flüssigem Gips aufgefüllt wird. Die Kanülen gelangen auf diese Weise in dem Sonderbehälter mit Gips umhüllt auf eine Deponie.

Da das manuelle Verbiegen der Kanülen die Gefahr von Verletzungen und von Kontakt mit infizierter Flüssigkeit mit sich bringt, ist es durch EP 0 136 392 B1 bekannt, die Kanülen unter Verwendung eines Stromflusses zu deformieren. Hierzu ist ein Gerät vorgesehen, bei dem unterhalb der Einführöffnung zwei federnd gelagerte, nicht miteinander in Kontakt stehende Elektroden angeordnet sind, die durch das Metallrohr der Kanüle verbunden werden können, so daß durch das Metallrohr der Kanüle ein Strom fließt. Die Einführöffnung ist so ausgebildet, daß die Kanüle einschließlich ihres Kunststoffkopfes in das Geräteinnere einführbar ist. Eine seitliche längliche Erstreckung der Einführöffnung erlaubt das Hintergreifen des Kopfes bei der seitlichen Bewegung in Richtung der länglichen Erstreckung, wobei eine Schrägführung auf der Unterseite der länglichen Erstreckung für eine Trennung der Kanüle von dem Spritzenkörper sorgt, so daß in eine Schublade in dem Gerät die komplette Kanüle mit dem deformierten Metallrohr hineinfällt und der Benutzer nur noch den unten offenen Spritzenkörper in der Hand behält.

Ein Gerät der eingangs erwähnten Art ist durch GB-A-2 211 420 bekannt. Unter einer Einführöffnung befindet sich eine durch zwei federnd gegeneinander gedrückte Rollen gebildete erste Elektrode, die durch das Metallrohr der Kanüle auseinandergedrückt werden und somit an dem eingeführten Metallrohr anliegen. Unterhalb der Einführöffnung befindet sich eine durch eine Platte oder einen Stutzen gebildete zweite Elektrode, auf die die Spitze des eingeführten Metallrohres trifft. An der Spitze schmilzt das Metallrohr durch den Stromfluß durch die beiden Elektroden und das dazwischen liegende Metallrohr wird durch ein weiteres Einführen zunehmend abgeschmolzen. Die Oberfläche der zweiten Elektrode ist horizontal ausgerichtet, so daß sich geschmolzenes Material des Metallrohres auf der Oberfläche ansammelt. Hierdurch soll an der Spitze des verbleibenden Restrohrstücks abgekühltes geschmolzenes Metallrohrmaterial haften, das das Metallrohr - und damit die Spritze - verschließt und ggf. gefährliche Inhaltsstoffe in der Spritze sicher einschließt.

Durch die EP-A-0 332 584 ist ein ähnliches Gerät bekannt, das zwei Einführöffnungen für unterschiedliche Kanülenlängen aufweist. Demgemäß befinden sich die zweiten Elektroden in unterschiedlichen Höhen relativ zu einer ersten flexiblen Elektrode, die an der Mantelfläche des Metallrohres der Kanüle zur Anlage kommt. Die zweite Elektrode ist durch einen Kohlenstoffblock gebildet und weist eine konvex gekrümmte Oberfläche auf. Die angestrebte Funktion dieses Geräts besteht darin, daß die Spitze der Kanülennadel durch Schmelzen eine sphärische Konfiguration erhält, die die Haut nicht mehr verletzt.

Der Erfindung liegt die Aufgabe zugrunde, ein Gerät der eingangs erwähnten Art hinsichtlich der Handhabung und Funktionssicherheit zu verbessern.

Diese Aufgabe wird erfindungsgemäß mit einem Gerät der eingangs erwähnten Art dadurch gelöst, daß der Auftreffpunkt der Spitze des Metallrohres während der gesamten Einführbewegung auf einer schrägen Abgleitfläche der zweiten Elektrode liegt.

Bei dem erfindungsgemäßen Gerät wird die Kanüle nur in ihrer Längsrichtung in die Einführöffnung des Gerätes eingeführt und weitgehend abgeschmolzen. Der dabei verbleibende Rest des Metallrohres weist ein abgeschmolzenes, d. h. nicht mehr scharfes und geschlossenes Ende auf. Bei dem Abschmelzvorgang entstehen aus dem Metallrohr spanartige bzw. tropfenartige Kanülenteile. Durch die Ausbildung der ersten Elektrode, die unmittelbar unterhalb der Einführöffnung vorgesehen ist, mit einer Durchtrittsöffnung für das Metallrohr wird ein zuverlässiger Stromfluß durch das Metallrohr erreicht, das mit der Spitze auf die zweite Elektrode auftrifft und dort schmilzt. Die zweite Elektrode ist am Auftreffpunkt mit einer zur Längsrichtung des Metallrohres schrägen Abgleitfläche versehen und als massiver, ortsfester Metallblock ausgebildet. Dadurch wird eine hohe Funktionssicherheit gewährleistet.

Durch die kreisrunde Ausbildung der Einführöffnung, die die Einführbewegung der Kanüle vorzugsweise durch einen Anschlag für den Kopf der Kanüle begrenzt, wird von einer Trennung der Kanüle von dem Spritzenkörper an dem Gerät abgesehen. Dadurch kann die abgeschmolzene Kanüle mit ihrem abgeschmolzenen und verschlossenen Ende an dem Spritzenkörper verbleiben und zusammen mit diesem entsorgt werden. Die verschlossene Kanüle bildet zusammen mit dem Spritzenkörper ein geschlossenes System, so daß etwaige infizierte Flüssigkeit sicher eingeschlossen bleibt, da ein Herausziehen des Spritzenkolbens in dem geschlossenen System einen Unterdruck erzeugen würde und daher praktisch nicht möglich ist. Etwaige, an dem Rest des Metallrohres befindliche infizierte Flüssigkeit wird durch den Stromfluß durch das Metallrohr desinfiziert, wobei die unmittelbar unterhalb der Einführöffnung angeordnete erste Elektrode dafür sorgt, daß praktisch das gesamte Metallrohr von dem Stromfluß erfaßt wird.

In einer vorteilhaften Ausführungsform ist die erste Elektrode mindestens zweiteilig ausgebildet, wobei die beiden Teile der ersten Elektrode zwischen sich die Durchtrittsöffnung bilden. Die beiden Teile sind dabei gegen die Rückstellkraft einer Feder zur Vergrößerung der Durchtrittsöffnung relativ zueinander bewegbar. Im Ruhezustand weist die Durchtrittsöffnung zweckmäßigerweise einen kleineren Durchmesser als übliche Metallrohre von Kanülen auf. Die relative Bewegung der beiden Teile der ersten Elektrode zueinander kann in einer einfachen konstruktiven Ausgestaltung dadurch erreicht werden, daß ein Teil der ersten Elektrode relativ zum anderen Teil schwenkbar gelagert ist.

Zur Erleichterung des Einführens ist die Einführöffnung zweckmäßigerweise durch einen Einfülltrichter gebildet, der gleichzeitig als Anschlag für den Kanülenkopf dienen kann.

Die Handhabbarkeit des erfindungsgemäßen Geräts wird dadurch noch erhöht, daß die Elektroden an eine im Gehäuse angeordnete Gleichstrombatterie anschließbar sind. Diese kann als aufladbare Batterie ausgebildet sein. In diesem Fall ist es zweckmäßig, wenn am Gehäuse ein Anschluß zum Aufladen der Gleichstrombatterie über ein Ladegerät vorgesehen ist.

Im Stromkreis kann ein Schalter für die Betriebsbereitschaft angeordnet sein, wobei zweckmäßig in einem parallelen Kreis eine Lampe zur Anzeige der Betriebsbereitschaft vorgesehen ist. Der Schalter ist willkürlich betätigbar und kann als Ein/Aus-Schalter ausgebildet sein. Nur, wenn er geschlossen ist, ist die Betriebsbereitschaft gegeben und es können Einweg-Kanülen entsorgt werden. In der geöffneten Stellung des Schalters ist der Stromkreis unterbrochen. Der Schalter ist zweckmäßig in dem Teil des Stromkreises angeordnet, der die Anode mit der Gleichstrombatterie verbindet. In einem parallelen Zweig des Stromkreises, der den Schalter und die Gleichstrombatterie einerseits bzw. die Kathode und Anode andererseits überbrückt, kann eine Lampe zur Anzeige der Betriebsbereitschaft vorgesehen sein. Diese Lampe brennt dann bei eingeschaltetem Schalter.

Die Erfindung wird anhand eines bevorzugten Ausführungsbeispiels weiter verdeutlicht und beschrieben. Es zeigen:
- Figur 1: - den Schaltplan des Geräts,
- Figur 2: - eine Draufsicht auf das Gerät von oben,
- Figur 3: - eine Seitenansicht des Geräts gemäß Figur 2,
- Figur 4: - eine Draufsicht auf das Gerät bei abgenommenem Gehäusedeckel und
- Figur 5: - eine Schnittdarstellung gemäß der Linie V-V in Figur 4 in vergrößernder Darstellung.

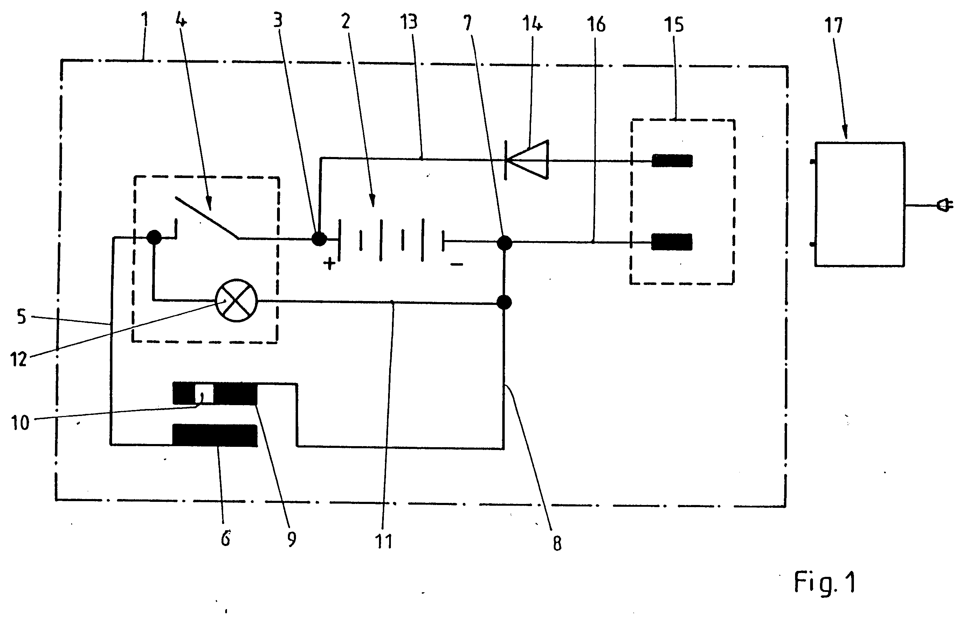

In Figur 1 ist das Gehäuse 1 des Geräts durch eine strichpunktierte Linie angedeutet und ansonsten der schematische Stromkreis wiedergegeben. In dem Gehäuse ist eine Gleichstrombatterie 2 untergebracht, deren Pluspol 3 mit einem Schalter 4 verbunden ist. Von dem Schalter 4 führt eine Leitung 5 zu einer Anode 6. Der Minuspol 7 der Gleichstrombatterie 2 ist über Leitung 8 mit einer Kathode 9 verbunden, die zweiteilig ausgebildet ist, wobei zwischen den beiden Teilen eine Durchtrittsöffnung 10 gebildet wird. In einem die Gleichstrombatterie 2 und den Schalter 4 überbrückenden Zweig 11 des Stromkreises ist eine Lampe 12 angeordnet. Wie ersichtlich, brennt die Lampe 12 nur bei geschlossenem Schalter 4.

Es handelt sich um eine wiederaufladbare Gleichstrombatterie 2, beispielsweise der Ausbildung 6 V, 6,5 Ah. Eine Leitung 13 führt vom Pluspol 3 über eine Entlade-Schutzdiode 14 zu einem Stecker bzw. einer Buchse 15. Ebenso führt vom Minuspol 7 eine entsprechende Leitung 16 zu dem Stecker 15, an den ein übliches Ladegerät 17, welches an eine Steckdose anschließbar ist, angeschlossen werden kann.

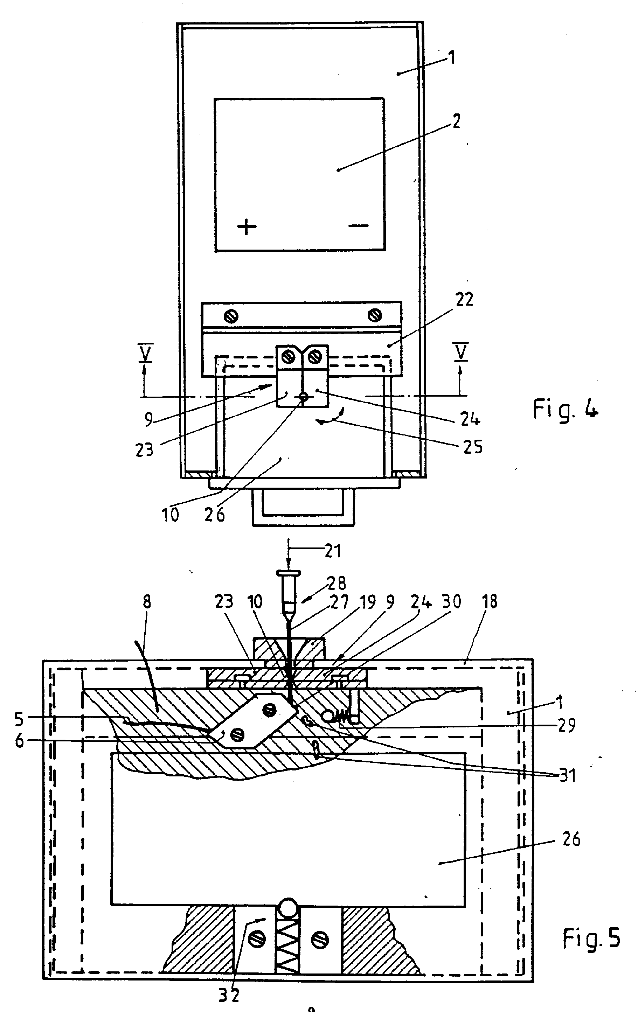

Es ist erkennbar, daß beim Hindurchstecken durch die Durchtrittsöffnung 10 der Kathode 9 bei geschlossenem Schalter 4 durch ein Metallrohr 27 einer Kanüle 28 der Stromkreis geschlossen wird. Anode 6 und Kathode 9 sind in einem bestimmten festgelegten Abstand zueinander angeordnet, so daß entsprechende Teile des Metallrohrs 27 der Kanüle 28 abgeschmolzen werden. Dies wird anhand von Figur 5 nachfolgend noch genauer erläutert.

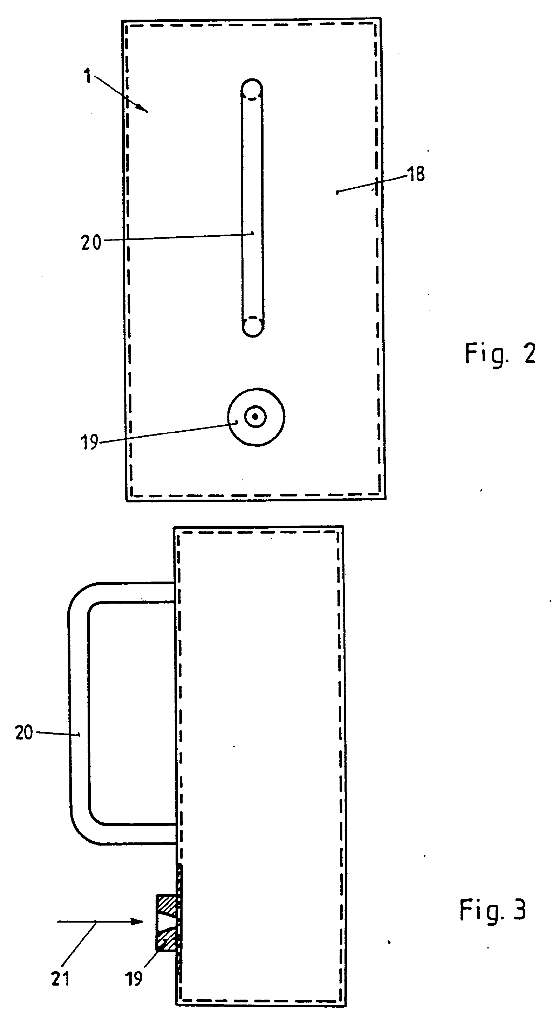

Figur 2 zeigt eine Draufsicht auf das Gerät mit seinem Gehäuse 1, welches zweckmäßig mehrteilig ausgebildet ist und einen nach oben abschließenden Gehäusedeckel 18 aufweist, auf den einerseits ein Einführtrichter 19 und andererseits ein Trage-griff 20 für das Gerät aufgesetzt sind. Durch die Gestaltung und Anordnung des Einführtrichters 19 wird eine Einführrichtung 21 vorgegeben, die bei auf einem Tisch stehenden Gerät beispielsweise vertikal von oben nach unten gerichtet ist.

Figur 4 zeigt eine Draufsicht bzw. eine Sicht in das Gerät von oben bei abgenommenen Gehäusedeckel 18. Man erkennt die Anordnung der Gleichstrombatterie 2. An einem Metallblock 22, der mit dem Gehäuse 1 verbunden sein kann, ist der feststehende Teil 23 und der im Sinne des Pfeils 25 schwenkbare Teil 24 der Kathode 9 dargestellt, wobei zwischen den beiden Teilen 23 und 24 die Durchtrittsöffnung 10 gebildet wird. Die Anode 6 ist aus Übersichtlichkeitsgründen in Figur 4 nicht dargestellt. Unterhalb der Einheit aus Kathode 9 und Anode 6 ist eine Schublade 26 herausziehbar am Gehäuse 1 vorgesehen, die zur Aufnahme abgebrannter Kanülenteile ausgebildet ist.

Figur 5 verdeutlicht die Arbeitsweise. Der Einführtrichter 19 ist mit Hilfe eines Gewindes in dem Gehäusedeckel 18 des Gehäuses eingeschraubt. Er besitzt eine trichterförmige Gestalt, dessen kleinster Durchmesser immer noch größer als der Außendurchmesser des Metallrohres 27 der Kanüle 28 ausgebildet ist. Durch die Anordnung des Einführtrichters 19 ist die Einführrichtung 21 vorgegeben, mit der die Kanüle 28 bis zu ihrem Kopf durch den Einführtrichter 19 in das Innere des Gehäuses 1 des Geräts abgesenkt wird. Man erkennt anhand von Figur 5 die beiden Teile 23 und 24 der Kathode 9 und den insoweit gebildeten Durchtrittsspalt 10, der kleiner als der Außendurchmesser des Metallrohrs 27 der Kanüle 28 ist. Über eine Feder 29 kann der schwenkbare Teil 24 der Kathode 9 gemäß Pfeil 25 (Figur 4) ausweichen, so daß die Durchtrittsöffnung 10 eine für das Hindurchtreten des Metallrohrs 27 der Kanüle 28 ausreichende Größe erhält. Wenn bei geschlossenem Schalter 4 eine Kanüle 28 in der beschriebenen Weise gemäß Einführrichtung 21 in das Gerät eingesteckt wird, gelangt die Spitze des Metallrohrs 27 auf die Anode 6, die als massiver Metallblock isoliert im Gehäuse 1 angeordnet und über die Leitung 5 mit der Gleichstrombatterie 2 verbunden ist. Die Anode 6 ist schräg zur Einführeinrichtung 21 angeordnet bzw. besitzt eine schräge Abgleitfläche 30, so daß beim Auftreffen der Spitze des Metallrohrs 27 der Stromkreis zwischen Kathode 9 und Anode 6 geschlossen wird. Dieser die Verbindung herstellende Teil des Metallrohrs 27 wird von einem Strom mit ca. 100 A durchflossen und dabei infolge seiner Dünnwandigkeit stark erhitzt und abgeschmolzen. Es bilden sich abgeschmolzene Kanülenteile 31, die in die darunter angeordnete Schublade 26 herabfallen und dort aufgefangen werden. Eine Kugelverrastung 32 sichert die Schublade 26 in der eingeschobenen Stellung, gestattet aber andererseits das Ausleeren der Schublade 26. Es versteht sich, daß beim Einschieben der Kanüle 28 in Richtung der Einführrichtung 21 durch das fortlaufende Abschmelzen der Kanülenteile 31 eine Zerstörung und Zerteilung des Metallrohrs 27 der Kanüle 28 fortlaufend stattfindet, bis der Einschiebevorgang durch das Aufsetzen des Kunststoffkopfes am Einführtrichter 19 beendet wird. Dabei ist die Dimensionierung so getroffen, daß auch der mit einem abgeschmolzenem Ende verbleibende Teil des Metallrohrs 27 unter eine ausreichende Hitzeeinwirkung gerät, so daß die Infektionsgefahr diesbezüglich beseitigt ist.

## Patentansprüche

1. Gerät zum Unschädlichmachen von zum einmaligen Gebrauch bestimmten Kanülen (28) einer Spritze, mit einem Gehäuse (1) mit einer kreisförmig ausgebildeten Einführöffnung (19), die nur die Einführung des Metallrohres (27) der Kanüle (28) in dessen Längsrichtung erlaubt, und einer unterhalb der Einführöffnung (19) angeordneten Elektrodenanordnung mit einer unmittelbar unterhalb der Einführöffnung (19) angeordneten ersten Elektrode (9) mit einer Durchtrittsöffnung (10) für das Metallrohr (27) und einer zweiten Elektrode (6), die als massiver Metallblock ausgebildet ist und auf die die Spitze des Metallrohres (27) nach dem Durchtritt durch die erste Elektrode (9) trifft und so einen Stromkreis zum Abschmelzen der jeweiligen Spitze des Metallrohres (27) schließt, wobei das Metallrohr (27) beim weiteren Einführen weiter abgeschmolzen wird und die Einführbewegung durch einen Anschlag der Einführöffnung (19) für einen Kopf der Kanüle (28) begrenzt wird, **dadurch gekennzeich****net, daß** der Auftreffpunkt der Spitze des Metallrohres (27) während der gesamten Einführbewegung auf einer schrägen Abgleitfläche (30) der zweiten Elektrode (9) liegt.

2. Gerät nach Anspruch 1, dadurch gekennzeichnet, daß die erste Elektrode (9) mindestens zweiteilig ausgebildet ist, daß zwei Teile (23, 24) der ersten Elektrode (9) zwischen sich die Durchtrittsöffnung (10) bilden und daß die beiden Teile (23, 24) gegen die Rückstellkraft einer Feder (29) zur Vergrößerung der Durchtrittsöffnung (10) relativ zueinander bewegbar sind.

3. Gerät nach Anspruch 2, dadurch gekennzeichnet, daß die Durchtrittsöffnung (10) der ersten Elektrode (9) im Ruhezustand einen kleineren Durchmesser als übliche Metallrohre (27) von Kanülen (28) aufweist.

4. Gerät nach Anspruch 2 oder 3, dadurch gekennzeichnet, daß ein Teil (24) der ersten Elektrode (9) relativ zum anderen Teil (23) schwenkbar gelagert ist.

5. Gerät nach einem der Ansprüche 1 bis 4, dadurch gekennzeichnet, daß die Einführöffnung (19) durch einen Einführtrichter gebildet ist.

6. Gerät nach einem der Ansprüche 1 bis 5, dadurch gekennzeichnet, daß die Elektroden (6, 9) an eine im Gehäuse (1) angeordnete Gleichstrombatterie (2) anschließbar sind.

7. Gerät nach Anspruch 6, dadurch gekennzeichnet, daß am Gehäuse (1) ein Anschluß (15) zum Aufladen der Gleichstrombatterie (2) über ein Ladegerät (17) vorgesehen ist.

8. Gerät nach einem der Ansprüche 1 bis 7, dadurch gekennzeichnet, daß lotrecht unter den Elektroden (6, 9) im Gehäuse (1) eine Schublade (26) zum Auffangen von abgeschmolzenen Kanülenteilen (31) angeordnet ist.

## Claims

1. Device for rendering disposable cannulae (28) of a syringe harmless, having a housing (1) with a circularly constructed introduction opening (19) which only permits the metal tube (27) of the cannula (28) to be introduced in the longitudinal direction thereof, and having an electrode arrangement, arranged below the introduction opening (19), with a first electrode (9), arranged directly below the introduction opening (19) and having a passage opening (10) for the metal tube (27), and a second electrode (6) which is constructed as a solid metal block and which is met by the tip of the metal tube (27) after passing through the first electrode (9) and thus makes a complete electrical circuit for melting the respective tip of the metal tube (27), the metal tube (27) being further melted on being further introduced and the introduction movement being limited by a stop of the introduction opening (19) for a head of the cannula (28), characterized in that the meeting point of the tip of the metal tube (27) lies on an oblique slide surface (30) of the second electrode (9) during the entire introduction movement.

2. Device according to Claim 1, characterized in that the first electrode (9) is constructed in at least two parts, in that two parts (23, 24) of the first electrode (9) form between them the passage opening (10), and in that to enlarge the passage opening (10) the two parts (23, 24) are movable relative to one another in opposition to the restoring force of a spring (29).

3. Device according to Claim 2, characterized in that the passage opening (10) of the first electrode (9) has in the rest condition a smaller diameter than conventional metal tubes (27) of cannulae (28).

4. Device according to Claim 2 or 3, characterized in that one part (24) of the first electrode (9) is mounted pivotally relative to the other part (23).

5. Device according to one of Claims 1 to 4, characterized in that the introduction opening (19) is formed by an introduction funnel.

6. Device according to one of Claims 1 to 5, characterized in that the electrodes (6, 9) are connectible to a direct current battery (2) arranged in the housing (1).

7. Device according to Claim 6, characterized in that a connection (15) for charging the direct current battery (2) by way of a charging device (17) is provided on the housing (1).

8. Device according to one of Claims 1 to 7, characterized in that a drawer (26) for collecting melted cannula parts (31) is arranged below the electrodes (6, 9) in the housing (1).

## Revendications

1. Appareil pour rendre inutilisables des canules (28), prévues pour une utilisation unique, d'une seringue, avec un boîtier (1) comportant une ouverture d'insertion (19) de forme circulaire, qui ne permet l'insertion du tube métallique (27) de la canule (28) que dans sa direction longitudinale, et comportant un agencement à électrodes disposé en dessous de l'ouverture d'insertion (19), avec une première électrode (9), disposée immédiatement en dessous de l'ouverture d'insertion (19) et comportant une ouverture de passage (10) pour le tube métallique (27), et une seconde électrode (6), qui est configurée en forme de bloc métallique massif, et avec laquelle la pointe du tube métallique (27) vient en contact après être passée à travers la première électrode (9), pour ainsi refermer un circuit de courant en vue de mettre chaque fois en fusion la pointe du tube métallique (27), tandis que le tube métallique (27) continue d'être fondu lorsque l'on poursuit l'insertion, le déplacement d'insertion étant limité par une butée de l'ouverture d'insertion (19) prévue pour une tête de la canule (28), caractérisé en ce que le point de contact de la pointe du tube métallique (27) repose sur une surface inclinée de glissement (30) de la seconde électrode (6) pendant la totalité du déplacement d'insertion.

2. Appareil selon la revendication 1, caractérisé en ce que la première électrode (9) est configurée en au moins deux pièces, en ce que deux pièces (23, 24) de la première électrode (9) forment entre elles l'ouverture de passage (10) et en ce que les deux pièces (23, 24) sont déplaçables l'une par rapport à l'autre, en opposition à la force de rappel d'un ressort (29), pour agrandir l'ouverture de passage (10).

3. Appareil selon la revendication 2, caractérisé en ce que l'ouverture de passage (10) de la première électrode (9) présente en position de repos un diamètre plus petit que les tubes métalliques habituels (27) de canules (28).

4. Appareil selon la revendication 2 ou 3, caractérisé en ce qu'une partie (24) de la première électrode (9) est maintenue à pivotement par rapport à l'autre partie (23).

5. Appareil selon l'une des revendications 1 à 4, caractérisé en ce que l'ouverture d'insertion (19) est configurée en forme d'entonnoir d'insertion.

6. Appareil selon l'une des revendications 1 à 5, caractérisé en ce que les électrodes (6, 9) peuvent être raccordées à une batterie à courant continu (2) disposée dans le boîtier (1).

7. Appareil selon la revendication 6, caractérisé en ce que sur le boîtier (1) est prévu un raccordement (15) en vue du chargement de la batterie à courant continu (2) par l'intermédiaire d'un appareil de charge (17).

8. Appareil selon l'une des revendications 1 à 7, caractérisé en ce que verticalement en dessous des électrodes (6, 9), un tiroir (26) est disposé dans le boîtier (1) pour réception des parties fondues (31) de canules.
